(19) Europäisches Patentamt
European Patent Office
Office européen des brevets

(11) Veröffentlichungsnummer: **0 384 199 A1**

(12) **EUROPÄISCHE PATENTANMELDUNG**

(21) Anmeldenummer: **90102186.5**

(51) Int. Cl.5: **C07D 209/48**

(22) Anmeldetag: **03.02.90**

(30) Priorität: **11.02.89 DE 3904082**

(43) Veröffentlichungstag der Anmeldung:
**29.08.90 Patentblatt 90/35**

(84) Benannte Vertragsstaaten:
**AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Freund, Wolfgang, Dr.**
**Johann-Gottlieb-Fichte-Strasse 71**
**D-6730 Neustadt(DE)**
Erfinder: **Schaefer, Bernd, Dr.**
**Hauptstrasse 22**
**D-6749 Dierbach(DE)**

(54) **Verfahren zur Herstellung von N-substituierten 3,4-5,6-Tetrahydrophthalimiden.**

(57) Verfahren zur Herstellung von N-substituierten 3,4,5,6-Tetrahydrophthalimiden der allgemeinen Formel I

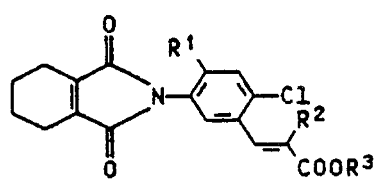

I

in der
$R^1$ für Wasserstoff oder Fluor steht,
$R^2$ Wasserstoff, Chlor, Brom oder eine $C_1$- bis $C_4$-Alkylgruppe ist
und in der $R^3$ eine $C_1$- bis $C_6$-Alkyl- oder eine $C_5$- bis $C_6$-Cycloalkylgruppe bedeutet, durch Umsetzung eines Aldehyds der allgemeinen Formel II

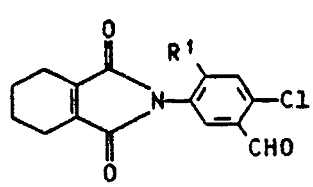

II

mit einem Phosphoran der allgemeinen Formel III

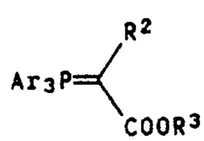

III

worin Ar einen unsubstituierten oder substituierten Phenylrest bedeutet, bei Temperaturen von -10 °C bis

EP 0 384 199 A1

100 °C und in Gegenwart eines Lösungsmittels.

## Verfahren zur Herstellung von N-substituierten 3,4,5,6-Tetrahydrophthalimiden

Die vorliegende Erfindung betrifft ein Verfahren zur Herstellung von N-substituierten 3,4,5,6-Tetrahydrophthalimiden der allgemeinen Formel I

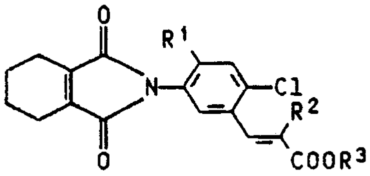

I

in der
$R^1$ für Wasserstoff oder Fluor steht,
$R^2$ Wasserstoff, Chlor, Brom oder eine $C_1$- bis $C_4$-Alkylgruppe ist
und in der $R^3$ eine $C_1$- bis $C_6$-Alkyl- oder eine $C_5$- bis $C_6$-Cycloalkylgruppe bedeutet.

N-substituierte 3,4,5,6-Tetrahydrophthalimide der allgemeinen Formel I sind aus EP-A 240 659 und aus der japanischen Offenlegungsschrift Nr. 155358/84 bekannt. Die in diesen Schriften beschriebenen Verfahren zur Herstellung von I gehen von nur umständlich zugänglichen und somit teuren Ausgangsmaterialien wie Phenylacetaldehydderivaten und Malonsäuremonoalkylestern aus. Weiterhin ist bei den bisher beschriebenen Verfahren die Synthese der Anilinderivate IV

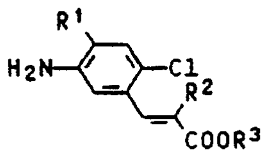

IV

aus den entsprechenden Nitroverbindungen von Nachteil. Eine selektive Reduktion der Nitrogruppe gelingt in der Regel nur mit Hilfe von Metallen wie Eisen oder mit Metallsalzen wie Zinn(II)chlorid in saurem, beispielsweise essigsaurem Milieu. Die Abtrennung der dabei anfallenden Metallsalze durch Extraktion oder Filtration ist schwierig und läßt sich nur unter hohem Aufwand bewerkstelligen - außerdem müssen hohe Kosten für die Entsorgung dieser Metallsalze und des sie enthaltenden Abwassers in Kauf genommen werden.

Der Erfindung lag deshalb die Aufgabe zugrunde, ein Verfahren zu finden, das die Herstellung der Verbindungen der allgemeinen Formel I aus leicht zugänglichen Ausgangsmaterialien und auf einfachere und kostengünstigere Weise erlaubt.

Dementsprechend wurde ein Verfahren zur Herstellung von N-substituierten 3,4,5,6-Tetrahydrophthalimiden der allgemeinen Formel I

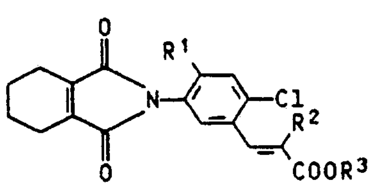

I

in der
$R^1$ für Wasserstoff oder Fluor steht,
$R^2$ Wasserstoff, Chlor, Brom oder eine $C_1$- bis $C_4$-Alkylgruppe ist
und in der $R^3$ eine $C_1$- bis $C_6$-Alkyl- oder eine $C_5$- bis $C_6$-Cycloalkylgruppe bedeutet, gefunden, das dadurch gekennzeichnet ist, daß man einen Aldehyd der allgemeinen Formel II

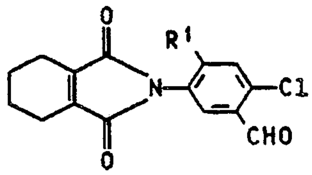

II

mit einem Phosphoran der allgemeinen Formel III

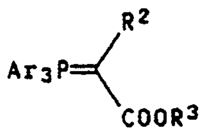

III

worin Ar einen unsubstituierten oder substituierten Phenylrest bedeutet, bei Temperaturen von -10 bis 100° C und in Gegenwart eines Lösungsmittels umsetzt.

Die als Ausgangsmaterial verwendeten Aldehyde der allgemeinen Formel II sind nach den Methoden der deutschen Patentanmeldung Nr. 3815042.5 auf einfache Weise zugänglich. Der Rest $R^1$ dieser Verbindungen kann für Wasserstoff oder Fluor stehen, bevorzugt steht er für Wasserstoff.

Die zur Herstellung der Tetrahydrophthalimide I benötigen Phosphorane III, welche auch als Phosphor-Ylide bezeichnet werden, sind nach literaturbekannten Methoden (z.B. Houben-Weyl, Methoden der Organischen Chemie, Bd. E1, S. 636-639, Georg-Thieme Verlag, Stuttgart 1982) erhältlich. Der Rest $R^2$ kann für Wasserstoff, Chlor, Brom oder eine $C_1$- bis $C_4$-Alkylgruppe stehen, bevorzugt steht $R^2$ für Chlor, Brom oder die Methylgruppe. Der Rest $R^3$ kann eine $C_1$- bis $C_6$-Alkyl- oder eine $C_5$- bis $C_6$-Cycloalkylgruppe sein. Die Alkylgruppen können verzweigt oder geradkettig sein. Bevorzugte Reste $R^3$ sind Alkylgruppen mit 1 bis 3 Kohlenstoffatomen.

Die Gruppen Ar dieser Phosphorane können unsubstituierte oder substituierte Phenylreste sein. Die Zahl der Substituenten pro Phenylrest sowie das Substitutionsmuster der Phenylreste sind in der Regel für das Gelingen des Verfahrens nicht kritisch, im allgemeinen trägt ein Phenylrest aber nicht mehr als 3 Substituenten. Als Substituenten der Phenylreste sind solche bevorzugt, welche sich unter den Bedingungen des erfindungsgemäßen Verfahrens inert verhalten, z.B. die Halogenide Fluor, Chlor und Brom, Alkylgruppen, bevorzugt $C_1$- bis $C_4$-Alkylgruppen, insbesondere die Methylgruppe, oder Alkoxygruppen, insbesondere die Methoxygruppe. Allerdings werden in der Regel Triarylphosphorane III mit unsubstituiertem Phenylrest bevorzugt im erfindungsgemäßen Verfahren eingesetzt.

Die Umsetzung der Ausgangsverbindungen II und III wird im allgemeinen vorteilhaft in Gegenwart eines Lösungsmittels durchgeführt. Als Lösungsmittel können alle üblicherweise bei der Durchführung von Wittig-Reaktionen verwendeten Lösungsmittel Anwendung finden, beispielsweise halogenierte Lösungsmittel, wie Chloroform, oder Ether, wie Tetrahydrofuran, Dioxan oder Ethylenyglykoldimethylether. Bevorzugte Lösungsmittel sind Alkohole, insbesondere $C_1$- bis $C_4$-Alkohole, wie Methanol, Ethanol, Propanol, Isopropanol, Butanol, Isobutanol oder tert.-Butanol. Die Lösungsmittel können auch in Form von Lösungsmittelgemischen eingesetzt werden, in der Regel werden jedoch bevorzugt die reinen Lösungsmittel verwendet. Dienen Alkohole als Lösungsmittel, so findet im allgemeinen vorteilhaft derjenige Alkohol als Lösungsmittel Verwendung, der der Alkoholkomponente der Estergruppe in I und II entspricht.

Es versteht sich von selbst, daß die optimale Reaktionstemperatur von den jeweils umzusetzenden Ausgangsverbindungen II und III sowie dem verwendeten Lösungsmittel abhängig ist. Im allgemeinen wird die Reaktion jedoch bei Temperaturen zwischen -10° C und 100° C, bevorzugt zwischen -10° C und 60° C und besonders vorteilhaft zwischen 10° C und 40° C ausgeführt.

Die Ausgangsverbindungen II und III können in stöchiometrischen Mengen miteinander umgesetzt werden. Es kann sich jedoch als vorteilhaft erweisen, wenn einer der beiden Reaktanten, II oder III, in einem molaren Überschuß von 10 bis 20 % in die Reaktion eingesetzt wird.

Die Umsetzung wird zweckmäßigerweise in Gegenwart von 0,3 bis 2 l, vorzugsweise von 0,5 bis 1 l Lösungsmittel pro Mol II vorgenommen.

Die Umsetzung kann diskontinuierlich in einem Rührreaktor oder kontinuierlich in einem Reaktionsrohr oder einer Rührreaktorkaskade erfolgen. Die Zugabereihenfolge der einzelnen Reaktanten ist dabei im allgemeinen nicht kritisch.

Der Verlauf der Reaktion kann mittels üblicher analytischer Methoden, wie Dünnschichtchromatographie oder Hochdruckflüssigchromatographie, verfolgt werden. Nach beendeter Reaktion kann das Produkt I nach

herkömmlichen Verfahren, wie Filtration, Zentrifugation oder durch Zugabe von Wasser mit anschließender Extraktion isoliert werden. Gewünschtenfalls können die so erhaltenen Tetrahydrophthalimide beispielsweise durch Umkristallisation oder mittels chromatographischer Methoden weiter gereinigt werden.

Nach dem erfindungsgemäßen Verfahren werden die Tetrahydrophthalimide I hinsichtlich der Alkenylseitenkette im allgemeinen als cis-trans-Isomerengemische erhalten, wobei in der Regel das trans-Isomere überwiegt.

Nach dem erfindungsgemäßen Verfahren werden die Tetrahydrophthalimide in guten Ausbeuten erhalten. Überraschenderweise reagiert das Phosphoran III offensichtlich nur mit der Aldehydcarbonylgruppe von II und nicht - wie es hätte erwartet werden müssen (vgl. Chem. Soc. Rev. 17, 1-30 (1988), insbesondere S. 21ff.) - mit der Imidcarbonylgruppe.

Die nach dem erfindungsgemäßen Verfahren erhältlichen N-substituierten 3,4,5,6-Tetrahydrophthalimide wirken zum Teil herbizid, zum Teil werden sie zu Herbiziden weiterverarbeitet (s. EP-A 240 659).

## Beispiele

### Beispiel 1

N-[5-(α-Bromacrylsäuremethylester)-4-chlor-2-fluor-phenyl]-3,4,5,6-tetrahydrophthalimid (1)

4,60 g (15 mmol) N-(2-Fluor-4-chlor-5-formyl-phenyl)-3,4,5,6-tetrahydrophthalimid und 6,80 g (16,5 mmol) Carbomethoxybrommethylen-triphenylphosphoran wurden in 15 ml Methanol 15 min lang bei Raumtemperatur gerührt. Die Reaktionsmischung wurde auf 0 °C gekühlt und das ausgefallene Produkt (1) abfiltriert.
Ausbeute: 70 % (1)
Fp. : 155 - 157 °C

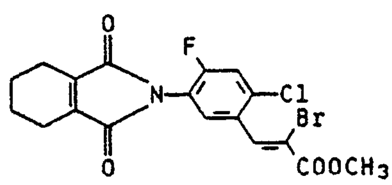

(1)

### Beispiel 2

N-[5-(α-Chloracrylsäureethylester)-4-chlor-2-fluor-phenyl]3,4,5,6-tetrahydrophthalimid (2)

4,60 g (15 mmol) N-(2-Fluor-4-chlor-5-formyl-phenyl)-3,4,5,6-tetrahydrophthalimid und 6,30 g (16,5 mmol) Carboethoxychlormethylen-triphenylphosphoran wurden in 15 ml Ethanol 15 min lang bei Raumtemperatur gerührt. Anschließend wurde auf 0 °C abgekühlt und das ausgefallene Produkt (2) abfiltriert.
Ausbeute: 68 % (2)
Fp. : 104 - 107 °C

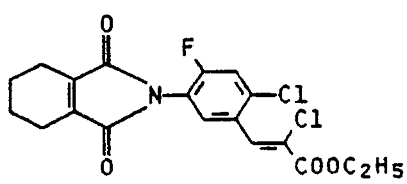

(2)

Beispiel 3

N-[3-(α-Chloracrylsäureethylester)-4-chlor-phenyl]-3,4,5,6-tetrahydrophthalimid (3)

Zu 956 g (2,5 mol) Carboethoxychlormethylen-triphenylphosphoran in 1,3 l Ethanol wurden bei 30°C 651 g (2,25 mol) N-(4-Chlor-5-formyl-phenyl)-3,4,5,6-tetrahydrophthalimid portionsweise eingetragen. Nach beendeter Zugabe wurde noch 30 min gerührt. Anschließend wurde auf -10° C abgekühlt und das ausgefallene Produkt (3) abfiltriert.
Ausbeute: 82 % (3)
Fp.: 112 - 114° C

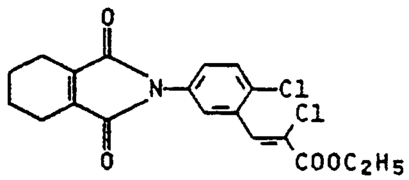

(3)

Beispiel 4

N-[3-(α -Bromacrylsäuremethylester)-4-chlor-phenyl]-3,4,5,6-tetrahydrophthalimid (4)

Zu 82,6 g (0,2 mol) Carbomethoxybrommethylen-triphenylphosphoran in 250 ml Methanol wurden bei 35°C 46,3 g N-(4-Chlor-5-formyl-phenyl)-3,4,5,6-tetrahydrophthalimid (0,16 mol) portionsweise eingetragen. Nach beendeter Zugabe wurde noch 30 min lang gerührt. Anschließend wurde auf -10°C abgekühlt und das ausgefallene Produkt (4) abfiltriert.
Ausbeute: 85 % (4)
Fp. : 151 - 154° C

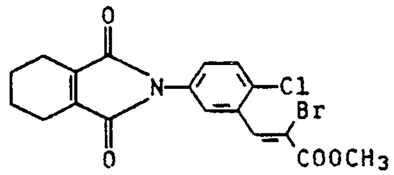

(4)

## Ansprüche

1. Verfahren zur Herstellung von N-substituierten 3,4,5,6-Tetrahydrophthalimiden der allgemeinen Formel I

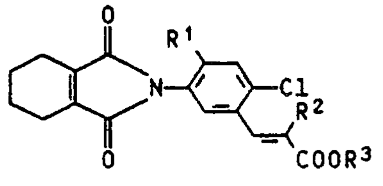

I

in der
$R^1$ für Wasserstoff oder Fluor steht,
$R^2$ Wasserstoff, Chlor, Brom oder eine $C_1$- bis $C_4$-Alkylgruppe ist,
und in der $R^3$ eine $C_1$- bis $C_6$-Alkyl- oder $C_5$- bis $C_6$-Cycloalkylgruppe bedeutet,

dadurch gekennzeichnet, daß man einen Aldehyd der allgemeinen Formel II

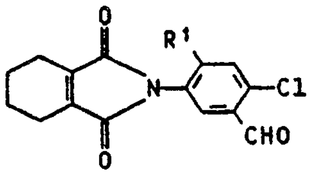

II

mit einem Phosphoran der allgemeinen Formel III

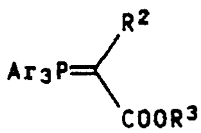

III

wobei Ar einen unsubstituierten oder substituierten Phenylrest bedeutet, bei Temperaturen von -10 bis 100°C und in Gegenwart eines Lösungsmittels umsetzt.

2. Verfahren nach Anspruch 1, dadurch gekennzeichnet, daß man als Lösungsmittel $C_1$- bis $C_4$-Alkohole verwendet.

Europäisches Patentamt

# EUROPÄISCHER RECHERCHENBERICHT

Nummer der Anmeldung

EP 90 10 2186

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-1 962 998 (SCHERING AG)<br>* Das ganze Dokument, insbesondere Seite 1 *<br>----- | 1 | C 07 D 209/48 |
| | | | **RECHERCHIERTE SACHGEBIETE (Int. Cl.5)** |
| | | | C 07 D 209/00 |

Der vorliegende Recherchenbericht wurde für alle Patentansprüche erstellt

| Recherchenort | Abschlußdatum der Recherche | Prüfer |
|---|---|---|
| DEN HAAG | 21-05-1990 | VAN BIJLEN H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)